# EUROPÄISCHE PATENTSCHRIFT

(11) **EP 4 545 027 B1**
(45) Veröffentlichungstag und Bekanntmachung des Hinweises auf die Patenterteilung: **01.10.2025**
(21) Anmeldenummer: 23206000.4
(22) Anmeldetag: 26.10.2023
(51) Int. Cl.: A61B 17/88

(54) **VORRICHTUNG ZUM HERAUSPRESSEN VON KNOCHENZEMENT, VERWENDUNG UND SYSTEM**
DEVICE FOR EXTRUDING BONE CEMENT, USE AND SYSTEM
DISPOSITIF POUR EXPRIMER DU CIMENT OSSEUX, UTILISATION ET SYSTÈME

(43) Veröffentlichungstag der Anmeldung: 30.04.2025
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- WO-A1-2012/066905
- US-A1- 2002 166 878
- US-A1- 2003 036 762
- US-A1- 2016 128 752
- US-A1- 2017 340 372

## Beschreibung

Die Erfindung betrifft eine Vorrichtung zum Herauspressen von Knochenzement aus einer Kartusche, eine Verwendung der Vorrichtung sowie ein System zum Herauspressen von Knochenzement.

Knochenzement wird üblicherweise durch Mischen eines Pulvers mit einer Flüssigkeit hergestellt. Beispielsweise sind Polymethylmethacrylat-(PMMA)-Knochenzemente bekannt, die aus einer flüssigen Monomerkomponente und einer Pulverkomponente zusammengesetzt sind. Die Monomerkomponente enthält im Allgemeinen das Monomer Methylmethacrylat und insbesondere einen darin gelösten Aktivator wie z. B. N,N-Dimethyl-p-toluidin. Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind und insbesondere einen Röntgenopaker und/oder den Initiator Dibenzoylperoxid auf. Beim Vermischen der Pulverkomponente mit der Monomerkomponente entsteht, z. B. durch Quellung der Polymere der Pulverkomponente im Methylmethacrylat, ein plastisch verformbarer Teig, der eigentliche Knochenzement (auch als Knochenzementteig bezeichnet). Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates kann sich die Viskosität des Zements erhöhen, bis dieser erstarrt.

PMMA-Knochenzemente werden hauptsächlich zur dauerhaften Fixierung von Gelenkendoprothesen im Knochen verwendet. Dabei werden im allgemeinen Zementmengen von 50 g bis 125 g oder mehr zur Fixierung einer Gelenkendoprothese verwendet. Der Knochenzement wird dabei während der Operation des Patienten benötigt und sollte entsprechend zügig bereitgestellt werden. Dies ist das wesentliche Anwendungsgebiet der vorliegenden Erfindung.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzement kommen, welche die mechanischen Eigenschaften des ausgehärteten Knochenzements negativ beeinflussen können. Andere Nachteile sind die Notwendigkeit, die Mengen manuell abzumessen und die nicht immer sichergestellte ausreichende Durchmischung.

Zur Vermeidung von Lufteinschlüssen im Knochenzement sind Vakuum-Zementiersysteme bekannt, beispielsweise US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1 und US 5 344 232 A. Hier besteht die Notwendigkeit, ein Vakuum anzulegen. Eine Weiterentwicklung in der Zementiertechnik stellen Zementiersysteme dar, in denen sowohl das Zementpulver als auch die Monomerflüssigkeit bereits in separaten Kompartimenten verpackt sind und unmittelbar vor der Zementapplikation im Zementiersystem miteinander vermischt werden. Solche geschlossenen Full-Prepacked-Mischsysteme oder ähnliche Systeme sind unter anderen in den Dokumenten EP 0 380 867 B1, EP 0 796 653 B1, EP 0 692 229 A1, DE 10 2009 031 178 B3, US 5 997 544 A, US 6 709 149 B1, WO 00/35506 A1 und EP 0 796 653 A2 beschrieben.

Mischvorrichtungen, bei denen eine externe Vorrichtung zum Herauspressen des Knochenzements verwendet wird, oder ähnliche Systeme sind in WO9416951A1, EP1741413B1, EP3054880B1 und DE19718648A1 offenbart. Mischvorrichtungen, bei denen ein Vakuum angelegt wird, um den Transport oder das Mischen einer Komponente oder des Knochenzements zu ermöglichen, oder ähnliche Systeme sind aus US8662736B2 und EP3093067B1 bekannt. EP2393456B1 beschreibt eine Mischvorrichtung, bei der die Flüssigkeit mittels eines Überdrucks in ein Pulver gedrückt wird.

Manuell zu bedienende Auspressvorrichtungen basieren beispielsweise auf manuell bewegbaren Hebelsystemen, die Schubstangen oder Zahnstangen antreiben und die mit den Zementkartuschen verbunden werden. Durch wiederholte Kippbewegung der Hebel wird die Schubstange oder Zahnstange in Richtung des Austragskolbens der Kartuschen bewegt, wobei der Austragskolben den Polymethylmethacrylat-Knochenzement durch Bewegung in Richtung Kartuschenkopf aus der Kartusche mit davor angebrachten Austragsrohren herauspresst. Derartige oder ähnliche Auspressvorrichtungen sind in US589344A, US5638997A und WO9416951A offenbart. Für den Austrag von kleinen Zementmengen bis zu ungefähr 10 g Knochenzement sind Schraubsysteme üblich, die Verwendung für die Stabilisierung von frakturierten Wirbelkörpern finden. Diese bisher bekannten Schraubsysteme sind mechanisch nur gering belastbar und nicht für den Austrag größerer Mengen von mindestens 50 g PMMA-Knochenzement geeignet. Exemplarisch für solche Austragsvorrichtungen oder ähnliche Vorrichtungen sind die Publikationen US6676663B2, US2012224452A1, CN218220290U und CN213190021U.

Die US 2002/166878 A1 beschreibt ein Hilfswerkzeug zum Drücken eines Kolbens, während eine Spritze oder ein ähnliches Instrument, das einen hochviskosen chemischen Wirkstoff enthält, fixiert wird, um den chemischen Wirkstoff herauszupressen, sowie ein Verfahren zum Herauspressen eines chemischen Wirkstoffs unter Verwendung desselben. Das Hilfsmittel zum Extrudieren eines chemischen Wirkstoffs umfasst einen Werkzeugkörper in Form eines zylindrischen Körpers mit einem darin ausgebildeten Raum zur Aufnahme eines röhrenförmigen Behälters, beispielsweise einer Spritze, der eine hochviskose Chemikalie enthält, beispielsweise Zement auf Kalziumphosphatbasis, der durch Kneten eines Kalziumphosphatpulvers und einer Knetflüssigkeit aus einer Polysaccharid-haltigen wässrigen Lösung hergestellt wird, und einen an seinem unteren Ende ausgebildeten Auslass. In dem zylindrischen Körper ist ein zylindrischer Raum ausgebildet, der an einer oberen Endfläche offen ist. Das Hilfswerkzeug weist außerdem einen Drücker mit einem Griffabschnitt zum Drücken eines in den röhrenförmigen Behälter einzusetzenden Kolbens und einen röhrenförmigen Körper auf, der über ein Schraubgewinde mit dem Werkzeugkörper verbunden ist und auf einer Seite des Griffabschnitts angeordnet ist, der auf den Kolben drückt. Der Griffabschnitt des Hilfsmittels zum Herauspressen des chemischen Wirkstoffs wird gedreht, um den röhrenförmigen Körper in den zylindrischen Raum zu drücken und den Kolben in den röhrenförmigen Behälter zu drücken, wodurch der chemische Wirkstoff aus dem röhrenförmigen Behälter herausgepresst wird.

Es ist die Aufgabe der Erfindung, einen besonders einfachen manuellen Austrag größerer Mengen an Knochenzement zu ermöglichen. Insbesondere soll eine besonders einfache und kostengünstige Vorrichtung geschaffen werden, die zumindest einige der Nachteile des Standes der Technik behebt.

Die Aufgabe wird gelöst durch die Vorrichtung zum Mischen von Knochenzement gemäß Anspruch 1 sowie die Verwendung und das System gemäß den nebengeordneten Ansprüchen. Vorteilhafte Ausgestaltungen sind in den Unteransprüchen angegeben.

Zur Lösung der Aufgabe dient eine Vorrichtung zum Herauspressen von Knochenzement aus einer Kartusche. Die Vorrichtung umfasst eine Gewindestange mit einem Außengewinde, einen Handgriff zum Drehen der Gewindestange sowie eine Adaptereinheit zum mechanischen Verbinden mit einer Kartusche für Knochenzement. Die Adaptereinheit weist eine Durchgangsöffnung mit einem Innengewinde für die Gewindestange auf. Erfindungsgemäß beträgt eine äußere Oberfläche eines Gewindegangs des Außengewindes höchstens 370 mm².

Es hat sich gezeigt, dass die äußere Oberfläche einen starken Einfluss auf die Reibungsverluste beim Drehen der Gewindestange in der Adaptereinheit und damit auf die für das Austragen des Knochenzements aufzubringende Kraft hat. Die äußere Oberfläche ist ein Maß für die Fläche des Außengewindes, die das Innengewinde kontaktiert. Deshalb ist die entstehende Reibung proportional zu der äußeren Oberfläche. Versuche haben gezeigt, dass es dabei unerheblich ist, ob eine Verringerung der äußeren Oberfläche durch eine Reduktion der Gewindetiefe, eine Erhöhung des Flankenwinkels, eine Reduktion des Durchmessers der Gewindestange, eine Reduktion der Steigung oder anderen oder durch Kombinationen von unterschiedlichen Maßnahmen erzielt wird. Maßgeblich ist lediglich der absolute Wert der äußeren Oberfläche.

Der Knochenzement ist in der Regel hochviskos. Daher sind häufig besonders große Kräfte notwendig, um den Knochenzement auszupressen. Für das Herauspressen von 130 g hochviskosem PMMA-Knochenzement aus einer Zementkartusche kann beispielsweise eine Vortriebskraft von bis zu 2 kN erforderlich sein, was der Gewichtskraft von etwa 200 kg entspricht. Deshalb ist eine Reduzierung der Reibung geboten, um den Knochenzement mit geringerer manueller Kraft auspressen zu können. Auf diese Weise können auch Personen mit geringerer Kraft die Vorrichtung ohne Schwierigkeiten bedienen. Dies ist insbesondere bedeutsam, da sich der Knochenzement mit fortschreitender Dauer nach dem Anmischen weiter verfestigt und daher im Laufe der Zeit die für das Auspressen benötigten Kräfte noch steigen. Versuche haben gezeigt, dass bei der beschriebenen maximalen äußeren Oberfläche eines Gewindegangs des Außengewindes für jeden Benutzer ein einfaches Austragen des Knochenzements durch manuelles Drehen des Handgriffs möglich ist. Bei größeren äußeren Oberflächen bewirken die Haft- und Gleitreibung ein sehr schwergängiges Austragen und damit eine schlechte Handhabbarkeit.

Die Vorrichtung kann aus Kunststoff oder Kunststoff mit Metall hergestellt sein. Eine komplizierte Mechanik wie im Falle einer Zementpistole ist nicht notwendig. Es werden keine Hebel, Bolzen, Stifte, Klemmungen, Rastelemente, Zahnstangen und/oder Zahnräder benötigt. Die für ein Herauspressen des Knochenzements notwendigen Kräfte sind geringer als die Kräfte, die mit herkömmlichen Vorrichtungen, beispielsweise Zementpistolen, benötigt werden.

Die erfindungsgemäße Vorrichtung ist einfach zu handhaben, da keine komplizierte Bedienung einer Pistole notwendig ist. Das Austragen erfolgt einfach durch Drehen des Handgriffs.

Ein Gewindegang meint eine Schraubenlinie des Gewindes über eine Strecke, die einer vollständigen relativen Drehung um 360° entspricht. Es wird also eine Umdrehung betrachtet. Die äußere Oberfläche ist die Fläche des Außengewindes, die das korrespondierende Innengewinde kontaktieren kann. Die äußere Oberfläche eines Gewindegangs setzt sich beispielsweise zusammen aus der Fläche einer ersten (beispielsweise aufsteigenden) Gewindeflanke, einer zweiten (beispielsweise absteigenden) Gewindeflanke, einer Fläche im Bereich der Erhebung des Gewindes, die sich beispielsweise als parallel zur Längsachse erstrecken kann und/oder sich zwischen den Gewindeflanken befinden kann, und einer weiteren Fläche im Bereich der Vertiefung bzw. des Tals des Gewindes, die sich zwischen zwei Gewindeflanken befinden kann. Diese vier Flächen werden über eine Umdrehung addiert. Es wird mit anderen Worten die Fläche zwischen zwei benachbarten Erhebungen oder Tälern des Gewindes betrachtet. Zwischen den einzelnen Flächen befindliche Rundungen oder Übergänge sind ebenfalls Teil der äußeren Oberfläche. Jeweilige Kennwerte für das Außengewinde der Gewindestange können entsprechend für das Innengewinde der Adaptereinheit gelten.

Im Fall eines Trapezgewindes entspricht die äußere Oberfläche beispielsweise den Flächen der beiden Gewindeflanken, der dazwischen liegenden, nach außen weisenden Fläche im Bereich der Erhebung des Gewindes, die den Außendurchmesser des Außengewindes definiert, sowie der nach außen weisenden Fläche im Bereich des Tals des Gewindes, die den Innendurchmesser des Außengewindes definiert.

Im Falle eines mehrgängigen Gewindes werden die Flächen aller Gänge des Gewindes über eine Umdrehung addiert.

Die Gewindestange ist dazu eingerichtet, durch manuelles Drehen am Handgriff gedreht zu werden. Insbesondere ist der Handgriff zu diesem Zweck drehfest mit der Gewindestange verbunden. Das Innengewinde wirkt mit dem Außengewinde der Gewindestange so zusammen, dass die Gewindestange in Bezug zum Innengewinde gedreht werden kann. Innen und Außengewinde korrespondieren. Durch das Zusammenwirken der Gewindestange mit der Adaptereinheit bewirkt das Drehen der Gewindestange eine axiale translatorische Relativbewegung zwischen Gewindestange und Adaptereinheit. Die Erstreckungsrichtung der Gewindestange definiert die axiale Richtung der Vorrichtung. Die Gewindestange ist bevorzugt durch die Durchgangsöffnung geführt, sodass das Innengewinde im Eingriff mit dem Außengewinde ist. Es können aber auch Gewindestange und Adaptereinheit separat vorliegen, sodass ein Benutzer vor dem bestimmungsgemäßen Gebrauch zunächst die Gewindestange in die Adaptereinheit eindrehen muss.

Die Adaptereinheit ist dazu eingerichtet, mechanisch an einer Kartusche mit Knochenzement befestigt zu werden. Insbesondere ist dazu eine drehfeste Verbindung zumindest in der Drehrichtung der Gewindestange vorgesehen, sodass das Drehen der Gewindestange die Befestigung der Adaptereinheit an der Kartusche nicht beeinträchtigt oder löst. Typischerweise weist die Adaptereinheit ein oder mehrere erste Befestigungsmittel auf und die Kartusche weist ein oder mehrere zweite Befestigungsmittel auf, wobei die beiden Befestigungsmittel dazu eingerichtet sind, zusammenzuwirken und auf diese Weise die mechanische Befestigung zu bewirken. Beispielsweise kann die Kartusche ein Außengewinde aufweisen und die Adaptereinheit kann ein korrespondierendes Innengewinde aufweisen. Die Durchgangsöffnung der Adaptereinheit ist insbesondere zentral angeordnet.

Ist die Adaptereinheit an der Kartusche befestigt, bewirkt die axiale Relativbewegung der Gewindestange in Bezug zur Adaptereinheit und damit zur Kartusche, dass das dem Handgriff gegenüberliegenden Ende der Gewindestange in die Kartusche eindringen kann und so Knochenzement aus der Kartusche herauspressen kann. Auf diese Weise kann aus dem der Adaptereinheit gegenüberliegenden Ende der Kartusche Knochenzement herausgepresst werden.

Insbesondere umfasst die Vorrichtung an dem Ende der Gewindestange, welches dem Handgriff gegenüberliegt, ein Druckelement zum Ausüben eines Drucks auf den in der Kartusche befindlichen Knochenzement. Dieser Druck kann direkt oder indirekt ausgeübt werden. Beispielsweise kann das Druckelement direkt auf den Knochenzement drücken oder auf ein weiteres Element wie z. B. einen Kolben der Kartusche drücken, das wiederum auf den Knochenzement drückt.

Erfindungsgemäß beträgt die äußere Oberfläche eines Gewindegangs des Außengewindes wenigstens 230 mm².

Die untere Grenze der äußeren Oberfläche bewirkt, dass die mechanische Stabilität der Gewindestange gewährleistet bleibt. Bei zu geringen äußeren Oberflächen sind beispielsweise die Gewindetiefe oder der Durchmesser der Gewindestange so gering, dass es zu einem Abscheren des Außengewindes und/oder zu einer Torsion bzw. einem Knicken der Gewindestange kommen kann.

Auch die Steigung des Außengewindes beeinflusst die für das Austragen benötigte Kraft. Die Steigung des Außengewindes ist der in axialer Richtung gemessene Weg, der bei einer Umdrehung zurückgelegt wird. Grundsätzlich führt eine zu geringe Steigung zu einer sehr hohen Übersetzung und damit zu einem erhöhten Aufwand bei der Benutzung, da eine größere Anzahl Drehungen zurückgelegt werden muss, um den Knochenzement auszutragen. Dies kann dazu führen, dass eine zu lange Zeit benötigt wird, was insbesondere während einer Operation und/oder bei einer fortschreitenden Verhärtung des Knochenzements unerwünscht ist. Grundsätzlich führt eine zu hohe Steigung zu einer geringen Übersetzung und damit zu einer sehr hohen Kraft, um den Knochenzement auszutragen. Zudem kann bei zu hohen Steigungen die Selbsthemmung verringert werden oder wegfallen, was wiederum die Bedienung unnötig verkompliziert. Die konkreten Zahlenwerte dieser Effekte sind von unterschiedlichen Rahmenbedingungen wie z. B. den verwendeten Materialien und Eigenschaften der jeweiligen Oberflächen abhängig.

Erfindungsgemäß beträgt eine Steigung des Außengewindes wenigstens 1 mm und höchstens 7 mm. Dies hat sich in Versuchen als besonders zielführend erwiesen. Insbesondere beträgt die Steigung wenigstens 2 mm, bevorzugt wenigstens 3 mm, besonders bevorzugt wenigstens 4 mm und höchstens 5 mm, bevorzugt höchstens 5 mm. In einer Ausführungsform beträgt die Steigung etwa oder genau 4,5 mm.

Erfindungsgemäß beträgt ein Außendurchmesser des Außengewindes der Gewindestange wenigstens 12 mm und höchstens 17 mm. Der Außendurchmesser meint den maximalen Durchmesser, der zwischen den zwei maximalen Erhebungen des Gewindegangs gemessen wird, also den Durchmesser eines das Außengewinde umgebenden, gedachten Kreiszylinders.

Insbesondere beträgt der Außendurchmesser wenigstens 13,5 mm und/oder höchstens 15 mm. In einer Ausführungsform beträgt der Außendurchmesser etwa oder genau 14 mm. Ein Außendurchmesser unterhalb von 12 mm ist nicht geeignet, den auftretenden Kräften bzw. Momenten in geeigneter Weise standzuhalten. Ein zu großer Außendurchmesser erhöht die Reibung und damit die für ein Austragen benötigte Kraft.

In einer Ausführungsform enthält das Innengewinde der Adaptereinheit wenigstens 2 und/oder höchstens 7 Gewindegänge. Es ist also die genannte Anzahl an Umdrehungen nötig, um die Gewindestange vollständig in das Innengewinde einzuschrauben. Grundsätzlich führt eine zu geringe Anzahl an Gewindegängen in der Adaptereinheit zu einem geringen Halt, zu einer geringen Festigkeit und/oder zu einer hohen Belastung des einzelnen Gewindegangs. Grundsätzlich führt eine zu hohe Anzahl an Gewindegängen zu einer erhöhten Reibung und daher zu einer großen benötigten Kraft für das Austragen des Knochenzements. Die genannten Zahlen haben sich als besonders zielführend erwiesen.

Insbesondere enthält das Innengewinde wenigstens 3 Gewindegänge, bevorzugt wenigstens oder genau 4 Gewindegänge und/oder höchstens 6 Gewindegänge, bevorzugt höchstens 5 Gewindegänge. Dies hat sich als Optimum aus den genannten Effekten erwiesen.

In einer Ausführungsform ist das Gewinde der Gewindestange ein Trapezgewinde. Dies hat sich als besonders geeignet erwiesen.

In einer Ausgestaltung weist das Außengewinde der Gewindestange eine Gewindetiefe von wenigstens 1 mm und/oder höchstens 4 mm auf. Grundsätzlich führt eine zu geringe Gewindetiefe zu einer verringerten Festigkeit und/oder zu einer erhöhten mechanischen Beanspruchung des Gewindes. Grundsätzlich führt eine zu große Gewindetiefe zu einer erhöhten Reibung und daher zu einer großen benötigten Kraft für das Austragen des Knochenzements. Die genannten Zahlen haben sich als besonders zielführend erwiesen.

Insbesondere beträgt die Gewindetiefe höchstens 2 mm und/oder ungefähr oder genau 1,5 mm. Dies hat sich hinsichtlich der genannten Effekte als optimal erwiesen.

In einer Ausgestaltung weist die Vorrichtung an dem Ende der Gewindestange, welches dem Handgriff gegenüberliegt, ein Druckelement zum Ausüben eines Drucks auf den in der Kartusche befindlichen Knochenzement auf. Insbesondere hat das Druckelement eine flache Oberfläche.

Das Druckelement kann wie beschrieben zum direkten oder indirekten Ausüben des Drucks eingerichtet sein. Beispielsweise kann das Druckelement als Teller ausgeführt sein. In einer Ausführungsform ist das Druckelement drehfest mit der Gewindestange verbunden. Beispielsweise kann das Druckelement mit der Gewindestange verschraubt, verschweißt oder mittels einer Steckverbindung mit der Gewindestange verbunden sein.

Das Druckelement kann ganz oder teilweise aus Metall oder Kunststoff, beispielsweise aus glasfaserverstärktem Kunststoff und/oder Polyamid, hergestellt sein.

In einer Ausgestaltung ist das Druckelement zweiteilig aufgebaut. Ein erster Teil des Druckelements ist drehfest mit der Gewindestange verbunden. Ein zweiter Teil des Druckelements ist um die Längsachse der Gewindestange drehbar mit dem ersten Teil verbunden.

Der zweite Teil dient dabei dem Ausüben des Drucks auf den Knochenzement bzw. auf ein Teil der Kartusche. Der erste Teil dient dem Anbinden des zweiten Teils an die Gewindestange und/oder dem Übertragen der Druckkraft der Gewindestange auf den zweiten Teil. Es kann zwischen dem ersten Teil des Druckelements ein Schmiermittel wie z. B. Silikon vorgesehen sein, um eine besonders leichtgängige relative Drehung zwischen dem ersten Teil und dem zweiten Teil zu gewährleisten. In einer Ausführungsform umgreift der zweite Teil den ersten Teil zumindest abschnittsweise oder umlaufend. Mit anderen Worten bildet der zweite Teil eine Hinterschneidung hinter dem ersten Teil. Auf diese Weise wird gewährleistet, dass der erste Teil und der zweite Teil sich nicht voneinander lösen. Eine axiale Bewegung des zweiten Teils weg vom ersten Teil und damit ein Verlieren des zweiten Teils wird so verhindert.

Insbesondere ist eine dem ersten Teil zugewandte Oberfläche des zweiten Teils und/oder eine dem zweiten Teil zugewandte Oberfläche des ersten Teils flach und/oder glatt ausgeführt. So wird eine besonders leichtgängige relative Drehung ermöglicht. Insbesondere ist eine dem ersten Teil zugewandte Oberfläche des zweiten Teils und/oder eine dem zweiten Teil zugewandte Oberfläche aus Metall hergestellt.

In einer Ausgestaltung weist die Adaptereinheit eine Ausnehmung auf. Insbesondere ist die Ausnehmung in axialer Richtung zwischen einem Verbindungselement oder einem Verbindungsbereich der Adaptereinheit zum mechanischen Verbinden mit der Kartusche der Adaptereinheit und dem Innengewinde bzw. der Durchgangsöffnung angeordnet. Die Ausnehmung ist dazu eingerichtet, das Druckelement zumindest im Wesentlichen aufzunehmen.

Insbesondere ist die Ausnehmung in Blickrichtung entlang der Längsachse der Gewindestange kreisrund geformt und/oder mittig angeordnet. Insbesondere ist die Ausnehmung kreiszylinderförmig. Die Ausnehmung dient dazu, das Druckelement aufzunehmen. So ragt das Druckelement nicht oder nicht wesentlich in Richtung des Verbindungselements über die Ausnehmung hinaus. Auf diese Weise wird gewährleistet, dass ein Verbindungsbereich der Kartusche, mit dem die Adaptereinheit beispielsweise auf eine Kartusche aufgesteckt werden kann, frei bleibt. Die Ausnehmung kann in axialer Richtung beispielsweise eine Erstreckung von wenigstens 3 mm, bevorzugt 4 mm und/oder höchstens 10 mm, insbesondere höchstens 7 mm, bevorzugt höchstens 5 mm, aufweisen. Die axiale Erstreckung der Ausnehmung kann ungefähr oder genau der axialen Erstreckung des Druckelements entsprechen. Das Druckelement kann vollständig in der Ausnehmung aufgenommen werden.

In einer Ausgestaltung umfasst die Gewindestange einen innenliegenden Stab, beispielsweise einen Metallstab, und/oder eine Ummantelung, beispielsweise eine Kunststoffummantelung.

Der innenliegende Stab führt zu einer mechanisch stabilen Gewindestange. Insbesondere ist der Stab nicht-rotationssymmetrisch geformt. So wird eine formschlüssige Verbindung zwischen dem Material des Stabes, z. B. Metall, und dem Material der Ummantelung, z. B. Kunststoff, gewährleistet. Beispielsweise kann der Stab sechseckig oder quadratisch sein. Insbesondere wird ein Metallstab mit oder aus einem Edelstahl wie beispielsweise 316L verwendet.

Insbesondere ist der Stab vollständig von Kunststoff umhüllt. Insbesondere besteht das Gewinde aus Kunststoff. Auf diese Weise wird ein besonders leichtgängiges Gewinde bereitgestellt. Der Stab wird vor äußeren Einflüssen geschützt.

In einer Ausführungsform weist der Metallstab einen Durchmesser von wenigstens 7 mm und/oder höchstens 10 mm, bevorzugt etwa oder genau 8 mm auf. Zu geringe Durchmesser können bei voller Belastung zu Torsion und/oder Ausknicken des Gewindestabs führen. Zu große Durchmesser erhöhen den Durchmesser des Gewindestabs und somit die Reibung.

In einer Ausgestaltung ist die Adaptereinheit dazu eingerichtet, durch einen Bajonettverschluss mit der Kartusche verbunden zu werden. Insbesondere entspricht eine Drehrichtung der Adaptereinheit zur Verbindung der Adaptereinheit mit der Kartusche einer Drehrichtung zum Einschrauben der Gewindestange in die Adaptereinheit.

Die Adaptereinheit umfasst demnach Verbindungselemente zur Herstellung eines Bajonettverschlusses mit der Kartusche. Die Kartusche umfasst insbesondere korrespondierende Verbindungselemente. Dies ermöglicht eine einfache und sichere Verbindung. Zudem ist ein Zusammenwirken mit bestehenden Kartuschen möglich.

Die Drehrichtung zum Einschrauben der Gewindestange in die Adaptereinheit ist die Drehrichtung, in der der Handgriff und damit die Gewindestange gedreht wird, um den Knochenzement aus der Kartusche herauszupressen. Während dieser Drehung wird die Verbindung somit in Richtung der verbundenen Stellung gedrückt. Es wird verhindert, dass ein Drehen zum Lockern oder Lösen der Verbindung führen kann.

In einer Ausgestaltung weist die Gewindestange eine Länge von wenigstens 15 cm und/oder höchstens 23 cm auf. In einer Ausgestaltung weist die Adaptereinheit einen Verbindungsbereich zum Aufstecken auf eine Kartusche auf. In einer Ausgestaltung weist der Verbindungsbereich einen Innendurchmesser von wenigstens 3,3 cm und/oder höchstens 4,0 cm auf.

Die Länge der Gewindestange beträgt insbesondere wenigstens 15 cm, bevorzugt wenigstens 17 cm, besonders bevorzugt wenigstens 19 cm und/oder höchstens 25 cm, bevorzugt höchstens 23 cm, besonders bevorzugt höchstens 21 cm oder 20 cm. Diese Länge ist besonders geeignet, die gewünschte Menge von beispielsweise wenigstens 50 g und/oder höchstens 130 g Knochenzement rasch und mit vertretbarem Kraftaufwand auszutragen.

Der Verbindungsbereich kann auf ein Ende der Kartusche aufgesteckt werden. Dies kann im Falle einer Schraubverbindung oder einer Bajonettverbindung beispielsweise durch ein Aufschrauben bzw. Aufdrehen realisiert werden. Der Verbindungsbereich kann einen Innendurchmesser von wenigstens 3 cm, bevorzugt wenigstens 3,3 cm, insbesondere wenigstens 3,5 cm und/oder höchstens 4,0 cm, insbesondere höchstens 3,8 cm aufweisen. Der Innendurchmesser des Verbindungsbereiches entspricht dabei im Wesentlichen dem Außendurchmesser der Kartusche. Es hat sich gezeigt, dass ein derartiger Durchmesser besonders geeignet ist, die gewünschte Menge von beispielsweise wenigstens 50 g und/oder höchstens 130 g Knochenzement rasch und mit vertretbarem Kraftaufwand auszutragen

In einer Ausgestaltung ist die Adaptereinheit, der Handgriff und/oder zumindest ein Teil der Gewindestange aus Kunststoff hergestellt. Insbesondere ist der Kunststoff ein Polyamid, Polyketon, Polysulfon und/oder ein Polyoxymethylen. Bevorzugt ist der Kunststoff Polyamid 6, Polyamid 6,6 und/oder Polyamid 12. Diese Kunststoffe haben sich hinsichtlich Festigkeit, Beständigkeit und Kosten als optimal erwiesen.

Ein weiterer Aspekt ist die Verwendung einer Vorrichtung nach einem der vorhergehenden Ansprüche zum Herauspressen einer Menge von wenigstens 50 g und/oder höchstens 130 g Knochenzement aus einer Kartusche. Alle Merkmale, Vorteile und Ausgestaltungen der oben genannten Vorrichtungen gelten auch für die Verwendung und umgekehrt.

Insbesondere wird PMMA-Knochenzement ausgepresst. Insbesondere erfolgt dies zur Fixierung einer Gelenkendoprothese im menschlichen Knochengewebe. Insbesondere ist die Kartusche eine Kartusche eines Zementiersystems für die Fixierung einer Gelenkendoprothese im menschlichen Knochengewebe.

Ein weiterer Aspekt der Erfindung ist ein System zum Herauspressen von Knochenzement, umfassend eine Kartusche für Knochenzement sowie eine erfindungsgemäße Vorrichtung zum Herauspressen von Knochenzement. Insbesondere enthält die Kartusche Knochenzement oder zumindest eine Komponente zur Herstellung von Knochenzement. Alle Merkmale, Vorteile und Ausgestaltungen der oben genannten Vorrichtung und der Verwendung gelten auch für das System und umgekehrt.

Insbesondere umfasst die Kartusche einen Verbindungsbereich, der mit dem Verbindungsbereich der Vorrichtung derart zusammenwirkt, dass eine mechanische Verbindung zwischen der Adaptereinheit und der Kartusche hergestellt werden kann.

Die Kartusche kann an dem der Adaptereinheit abgewandten Ende mit einem oder mehreren geeigneten Schläuchen und/oder einem oder mehren geeigneten Rohren verbunden werden, um den Knochenzement an die gewünschte Stelle zu befördern. Auch ein Befüllen von einer oder mehreren Gießformen für Spacer mit Knochenzement ist möglich.

Ein Verfahren zum Herauspressen von Knochenzement aus einer Kartusche wird ebenso beschrieben (nicht beansprucht), bei dem der Handgriff einer erfindungsgemäßen Vorrichtung manuell gedreht wird, so dass Knochenzement aus der Kartusche herausgepresst wird. Alle Merkmale, Vorteile und Ausgestaltungen der oben genannten Vorrichtung, der Verwendung und das System gelten auch für das Verfahren und umgekehrt.

Nachfolgend werden Ausführungsbeispiele der Erfindung auch anhand von Figuren näher erläutert.

Es zeigen:
- Figur 1:: eine perspektivische Ansicht einer erfindungsgemäßen Vorrichtung mit einer alternativen Ausgestaltung einer Adaptereinheit,
- Figur 2:: eine Schnittdarstellung einer erfindungsgemäßen Vorrichtung,
- Figur 3:: eine weitere Schnittdarstellung einer erfindungsgemäßen Vorrichtung,
- Figur 4:: eine weitere Schnittdarstellung einer erfindungsgemäßen Vorrichtung,
- Figur 5:: eine Schnittdarstellung der Verwendung einer erfindungsgemäßen Vorrichtung,
- Figur 6:: eine weitere Schnittdarstellung der Verwendung einer erfindungsgemäßen Vorrichtung, sowie
- Figur 7:: ein vergrößertes Detail einer Gewindestange.

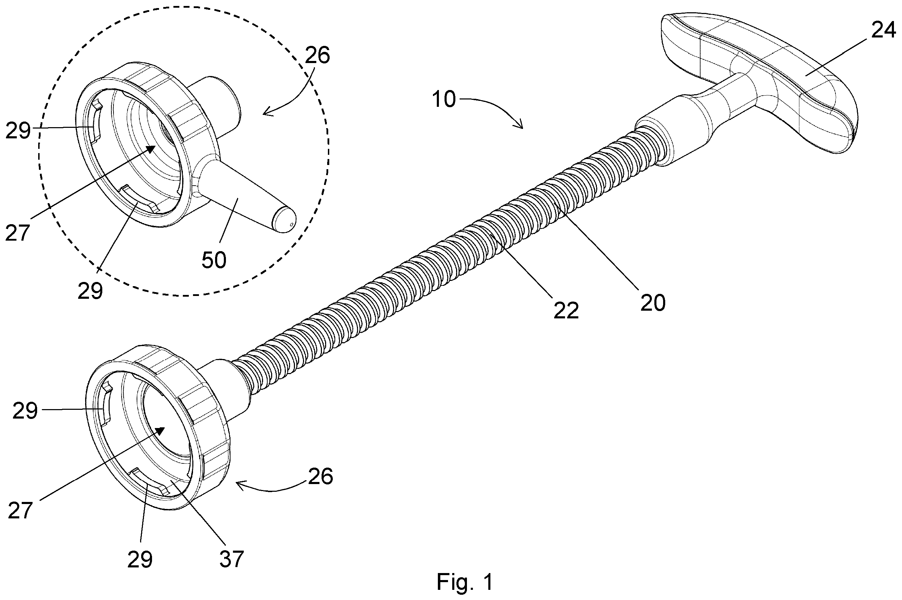

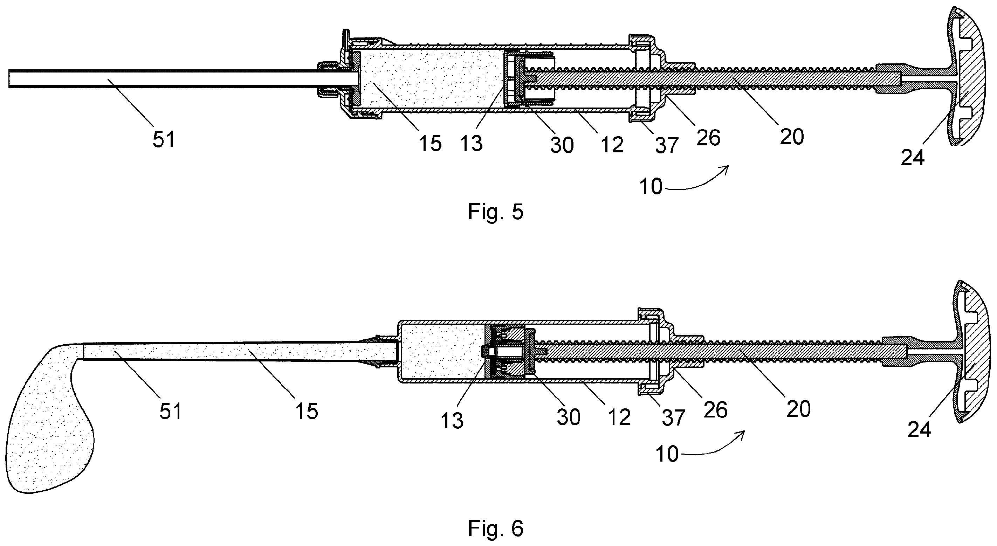

Figur 1 zeigt eine erfindungsgemäße Vorrichtung 10 zum Herauspressen von Knochenzement aus einer Kartusche. Die Vorrichtung 10 umfasst eine Adaptereinheit 26 mit einem Verbindungsbereich 37 zum mechanischen Verbinden mit einer Kartusche. Der Verbindungsbereich 37 kann in der hier gezeigten Ausführungsform auf einen entsprechenden Bereich einer Kartusche aufgesteckt bzw. aufgedreht werden, wie dies in den Figuren 5 und 6 dargestellt ist. In der hier gezeigten Ausführungsform hat die Adaptereinheit 26 Verbindungselemente 29 zur Herstellung einer Bajonettverbindung. Die Drehrichtung zum Befestigen der Bajonettverbindung entspricht dabei der Drehrichtung zum Einschrauben der Gewindestange 20.

Die Vorrichtung umfasst ferner eine Gewindestange 20 mit einem Außengewinde 22. Die Gewindestange 20 ist durch die Durchgangsöffnung 27 hindurchgeführt, sodass das Außengewinde 22 mit dem in der Durchgangsöffnung 27 befindlichen Innengewinde im Eingriff ist. An der rechts dargestellten Seite der Gewindestange 20 befindet sich der manuell bedienbare Handgriff 24 zum Drehen. Wird die Gewindestange 20 gedreht, erfolgt eine axiale Bewegung der Gewindestange durch die Adaptereinheit 26, sodass der Knochenzement herausgepresst werden kann.

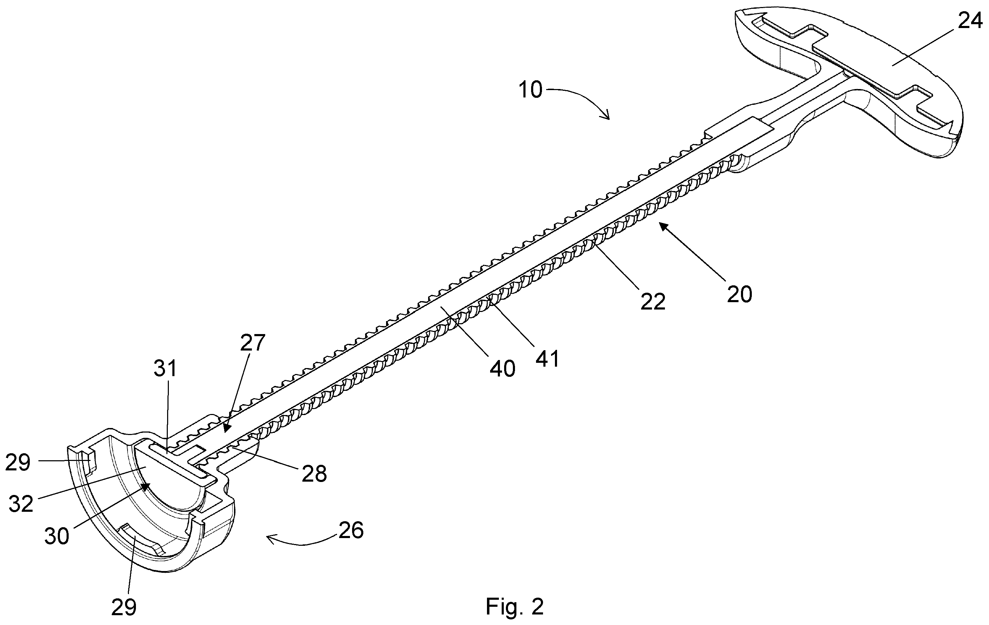

An der dem Handgriff 24 gegenüberliegenden Seite der Gewindestange 20 ist die Gewindestange 20 zum Ausüben eines Drucks bzw. einer Kraft auf den Knochenzement eingerichtet. Dazu kann ein Druckelement vorhanden sein, beispielsweise das in Figur 2 dargestellte Druckelement 30.

In einem gestrichelten Kreis ist eine alternative Adaptereinheit 26 dargestellt. Diese ist grundsätzlich so ausgestaltet wie die darunter dargestellte und oben beschriebene Adaptereinheit 26, umfasst jedoch zusätzlich ein Griffteil 50. Dieses kann die Form eines in radialer Richtung abstehenden Dorns aufweisen. Das Griffteil 50 dient als Widerlager beim manuellen Drehen des Handgriffs 24. Der Benutzer kann eine Hand zum Drehen am Handgriff 24 nutzen und gleichzeitig mit der anderen Hand die Adaptereinheit 26 bzw. die damit verbundene Kartusche am Griffteil 50 fixieren. So ist eine geringere Haltekraft und damit ein leichteres Drehen möglich. Das Austragen von Knochenzement wird weiter vereinfacht.

Figur 2 zeigt eine ähnliche Ausgestaltung der Vorrichtung 10 wie Figur 1 in einer entlang der Mittellängsachse der Gewindestange 20 geschnittenen Darstellung. Um Dopplungen zu vermeiden, wird lediglich auf die Unterschiede zur Figur 1 eingegangen.

Es ist ersichtlich, dass an dem Ende der Gewindestange 20, welches dem Handgriff 24 gegenüberliegt, ein Druckelement 30 angeordnet ist. Das Druckelement 30 dient der direkten oder indirekten Ausübung eines Drucks auf Knochenzement, um diesen aus einer Kartusche herauszupressen. Beispielsweise kann das Druckelement 30 einen Kolben der Kartusche bewegen, welcher wiederum den Knochenzement aus der Kartusche herauspresst.

Das Druckelement 30 ist zweiteilig aufgebaut. Ein erster Teil 31 ist fest mit der Gewindestange 20 verbunden, dreht sich also bei einer Drehung der Gewindestange 20 gemeinsam mit dieser. Ein zweiter Teil 32 ist drehbar mit dem ersten Teil 31 verbunden, so dass der zweite Teil 32 sich beim Drehen der Gewindestange 20 nicht mit dreht. Auf diese Weise wird keine Drehbewegung, sondern lediglich eine axiale Druckkraft auf den Knochenzement 15 bzw. den Kolben ausgeübt. Die einander kontaktierenden Oberflächen des ersten Teils 31 des zweiten Teils 32 sind bevorzugt eben und glatt ausgeführt, um eine leichtgängige Drehung zu ermöglichen.

Zudem zeigt sich, dass die Gewindestange 20 einen innenliegenden Kern, im hier gezeigten Beispiel einen Metallstab 40, sowie eine Ummantelung, im hier gezeigten Beispiel eine Kunststoffummantelung 41 aufweist. Der Metallstab 40 verläuft zentral im Inneren der Gewindestange 20 und trägt maßgeblich zur mechanischen Stabilität der Gewindestange 20 bei. Die Kunststoffummantelung 41 bildet das Außengewinde 22 aus und schützt den Metallstab 40 vor äußeren Einflüssen. Der Metallstab 40 ist bevorzugt nicht-rotationssymmetrisch ausgebildet, um eine drehfeste Verbindung zum Handgriff 24 zu gewährleisten.

In Figur 2 ist zudem das Innengewinde 28 an der Durchgangsöffnung 27 der Adaptereinheit 26 dargestellt. Das Innengewinde enthält 4 oder 5 Gewindegänge. Das Außengewinde 22 wie auch das Innengewinde 28 hat eine Steigung zwischen 3 mm und 6 mm.

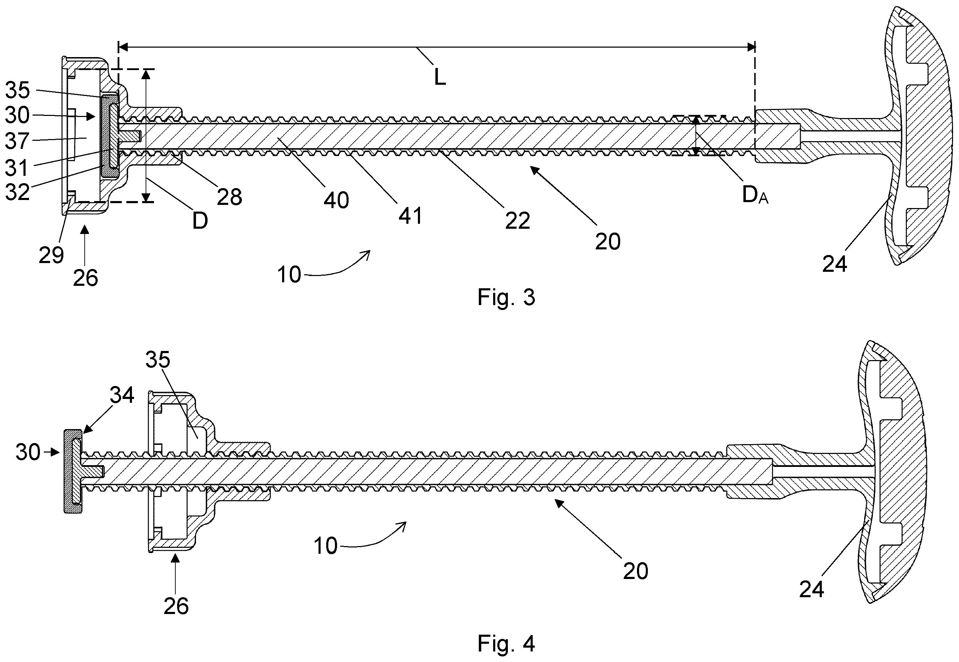

Die Figuren 3 und 4 zeigen ähnliche Ausgestaltungen der Vorrichtung 10 wie Figur 2 als Schnittzeichnung entlang der Mittellängsachse. Auch hier wird lediglich auf die zusätzlichen Merkmale eingegangen, um Dopplungen zu vermeiden. In Figur 3 ist wie in den Figuren 1 und 2 eine vollständig herausgeschraubte Position der Gewindestange 20 aus der Adaptereinheit 26 dargestellt. Dagegen ist in Figur 4 eine Position gezeigt, in der die Gewindestange 20 bereits teilweise durch die Adaptereinheit 26 hindurchgedreht ist, wie dies beim Herauspressen von Knochenzement erfolgt.

Es ist dargestellt, dass die Adaptereinheit 26 eine Ausnehmung 35 aufweist, in welche das Druckelement 30, wie in Figur 3 gezeigt, vollständig aufgenommen werden kann. Auf diese Weise verbleibt der auf der linken Seite dargestellte Verbindungsbereich 37 der Adaptereinheit 26 frei von dem Druckelement 30. So kann der Verbindungsbereich 37 über das zugehörige Ende der Kartusche gestülpt bzw. auf dieses aufgesteckt oder aufgeschraubt werden, ohne dass dabei das Druckelement 30 stört.

In Figur 3 sind zudem einige Maße der Vorrichtung 10 dargestellt. Die Gewindestange 20 hat eine Länge L zwischen 18 cm und 20 cm. Die Länge L wird zwischen den äußersten Bereichen gemessen, in denen das Außengewinde 22 vorliegt. Der Verbindungsbereich 37 der Adaptereinheit 26 hat einen Durchmesser D zwischen 34 mm und 40 mm. Der Durchmesser des Verbindungsbereichs 37 entspricht ungefähr dem Außendurchmesser des korrespondierenden Verbindungsbereichs der anzuschließenden Kartusche.

Es ist ersichtlich, dass der zweite Teil 32 des Druckelements 30 den ersten Teil 31 des Druckelements 30 umgreift bzw. mit diesem eine Hinterschneidung 34 ausbildet. Auf der rechten Seite erstreckt sich die insbesondere umlaufende, äußere Krempe des zweiten Teils 32 nach innen und hintergreift den radial äußeren Rand des ersten Teils 31.

Der erste Teil 31 und/oder der zweite Teil 32 kann in Form eines Tellers ausgeführt sein. Der zweite Teil 32 kann einen Durchmesser von wenigstens 20 mm und/oder höchstens 30 mm, beispielsweise ungefähr oder genau 25 mm aufweisen.

Zudem ist der Außendurchmesser D_{A} der Gewindestange 20 dargestellt, der in radialer Richtung zwischen den Erhebungen des Außengewindes 22 gemessen wird. Der Außendurchmesser D_{A} beträgt im hier gezeigten Beispiel zwischen 13,5 mm und 15 mm.

Die Figuren 5 und 6 zeigen die Verwendung von erfindungsgemäßen Vorrichtungen 10 mit unterschiedlichen Kartuschen 12. Der Verbindungsbereich 37 der Adaptereinheit 26 ist jeweils, wie beschrieben, auf ein Ende der Kartusche 12 gestülpt und an diesem mechanisch befestigt. Die Kartusche 12 weist an dem gegenüberliegenden Ende eine Auslassöffnung auf, an die beispielsweise ein Rohr 51 zum Austragen des Knochenzements 15 angeschlossen ist. Im Inneren der Kartusche befindet sich der Knochenzement 15. Typischerweise umfasst die Kartusche 12 zudem einen Kolben 13, welcher gegenüber der Innenwand der Kartusche 12 zumindest im Wesentlichen abgedichtet ist. Das Druckelement 30 der Vorrichtung 10 ist beispielsweise dazu eingerichtet, ein axialen Druck auf diesen Kolben 13 auszuüben, um den Knochenzement 15 aus der Kartusche herauszupressen. Figur 5 zeigt dabei einen beginnenden Auspressvorgang, bei dem das Druckelement 30 und der Kolben 13 so weit nach links gedrückt worden sind, dass der Knochenzement 15 flächig am Kolben 13 anliegt. Figur 6 zeigt einen weiter fortgeschrittenen Auspressvorgang, bei dem durch fortgeführtes Drehen am Handgriff 24 Knochenzement 15 durch das Rohr 51 hindurch und aus diesem heraus befördert worden ist.

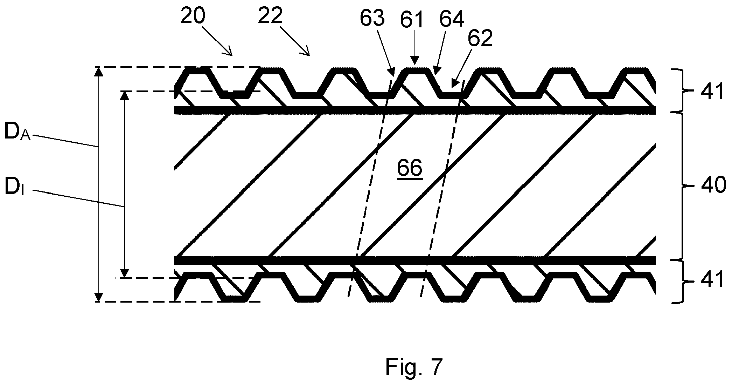

Figur 7 zeigt einen vergrößerten Ausschnitt eines Schnitts durch eine Gewindestange 20. Diese umfasst einen innenliegenden Metallstab 40 und eine Kunststoffummantelung 41, die den Metallstab 40 vollständig umschließt. Es ist der Außendurchmesser D_{A} der Gewindestange 20 dargestellt, der dem maximalen Durchmesser zwischen den beidseitigen Erhebungen des Außengewindes 22 entspricht. Zudem ist der Innendurchmesser D_{I} der Gewindestange 20 dargestellt, der dem minimalen Durchmesser zwischen den beidseitigen Vertiefungen oder Tälern des Außengewindes 22 entspricht. Das Gewinde hat eine Gewindetiefe zwischen 1 mm und 2 mm. Die Differenz zwischen Innendurchmesser D_{I} und Außendurchmesser D_{A} beträgt also zwischen 2 mm und 4 mm.

Ein Gewindegang 66 ist exemplarisch durch gestrichelte Linien abgegrenzt. Der Gewindegang entspricht einer vollständigen Drehung des Außengewindes 22 in Bezug zu dem korrespondierenden Innengewinde. Die Gewindestange 20 ist so eingerichtet, dass eine äußere Oberfläche eines Gewindegangs 66 des Außengewindes 22 höchstens 370 mm² beträgt. Die äußere Oberfläche des Gewindes bestimmt sich im hier gezeigten Beispiel eines Trapezgewindes als Summe aus der oberen Fläche 61, die der Fläche im Bereich der Erhebung des Gewindes entspricht, der unteren Fläche 62, die der Fläche im Bereich der Vertiefung des Gewindes entspricht, der ersten (aufsteigenden) Flankenfläche 63 sowie der zweiten (absteigenden) Flankenfläche 64.

In nicht beanspruchten Beispielen beträgt eine äußere Oberfläche eines Gewindegangs des Außengewindes höchstens 450 mm², insbesondere höchstens 430 mm², bevorzugt höchstens 400 mm². Erfindungsgemäß beträgt eine äußere Oberfläche eines Gewindegangs des Außengewindes höchstens 370 mm². In einer Ausgestaltung beträgt eine äußere Oberfläche eines Gewindegangs des Außengewindes höchstens 350 mm², insbesondere höchstens 330 mm², bevorzugt höchstens 300 mm². In nicht beanspruchten Beispielen beträgt eine äußere Oberfläche eines Gewindegangs des Außengewindes mindestens 100 mm², typischerweise mindestens 130 mm², insbesondere mindestens 160 mm², bevorzugt mindestens 180 mm², besonders bevorzugt mindestens 200 mm² und weiter bevorzugt mindestens 215 mm². In einer Ausgestaltung beträgt eine äußere Oberfläche eines Gewindegangs des Außengewindes mindestens 250 mm², typischerweise mindestens 280 mm², insbesondere mindestens 320 mm² und bevorzugt mindestens 350 mm². Diese Werte können je nach Ausgestaltung und Materialien besonders geeignet sein.

### Beispiel 1:

Es wurde eine Vorrichtung mit einer Steigung des Gewindes von 4,5 mm, einem Außendurchmesser D_{A} des Außengewindes von 14 mm und einer Gewindetiefe von 1,5 mm hergestellt. Dies hat sich als optimal erwiesen, um mit geringem Kraftaufwand zügig bis zu 130 g hochviskosen Knochenzement aus Zementkartuschen auszupressen. Ein kompletter Austrag von 130 g Knochenzement aus einer handelsüblichen Zementkartusche PALAMIX^{®} der Anmelderin konnte innerhalb von 1,5 Minuten erreicht werden. Ähnliche Ergebnisse wurden mit dem geschlossenen Zementiersystem PALACOS PRO^{®} (Version von 2021) der Anmelderin erreicht.

### Beispiel 2:

Es wurden Adaptereinheiten 26, Gewindestangen 20 mit einem Durchmesser von 30 mm, 25 mm, 20 mm und 14 mm und einer Steigung von 4,5 mm und 7,0 mm und dazu passende Handgriffe mit selektivem Lasersintern (SLS) aus Polyamid 12 gefertigt. Insbesondere werden einige oder alle Komponenten chemisch nachgeglättet. Die Gewindestange 20 enthält im Inneren eine handelsübliche Edelstahl-Sechskantstange. Insbesondere wurde diese nach der Herstellung des Kunststoffteils in das Kunststoffteil eingepresst.

Für die Auspressversuche wurden kommerziell erhältliche Mischsysteme PALAMIX und der Polymethylmethacrylat-Knochenzement PALACOS R+G der Anmelderin sowie handelsübliches Vancomycinhydrochlorid eingesetzt.

Es wurden Auspressversuche mit jeweils zwei Packungen PALACOS R+G (2 x 40,8 g Zementpulver plus 2 x 18,5 g Monomerflüssigkeit) in einer PALAMIX-Kartusche angemischt, die rund 118 g Knochenzement ergeben. Anschließend wurde mit den hergestellten Vorrichtungen der Knochenzement ausgepresst und die Handhabbarkeit der jeweiligen Vorrichtungen beurteilt. In der nachfolgenden Tabelle sind die Ergebnisse dargestellt.

| Außendurchmesser des Gewindes [mm] | Steigung des Gewindes [mm] | Äußere Oberfläche eines Gewindegangs [mm²] | Leichte Auspressbarkeit |
|---|---|---|---|
| 30 | 7,0 | 769 | nein |
| 25 | 7,0 | 632 | nein |
| 20 | 7,0 | 495 | nein |
| 14 | 7,0 | 331 | ja |
| 30 | 4,5 | 556 | nein |
| 25 | 4,5 | 459 | nein |
| 20 | 4,5 | 361 | ja |
| 14 | 4,5 | 244 | ja |

Weiterhin wurden analoge Versuche mit jeweils zwei Packungen PALACOS R+G und zwei Monomerampullen mit jeweils 18,5 g Monomerflüssigkeit durchgeführt, denen jeweils 4,0 g Vancomycinhydrochlorid zugesetzt wurde. Die theoretische Zementmenge betrug rund 126 g Zementteig. Es wurden analoge Ergebnisse zu der ersten Versuchsreihe erzielt.

**Bezugszeichenliste**

| | |
|---|---|
| Vorrichtung | 10 |
| Kartusche | 12 |
| Kolben | 13 |
| Knochenzement | 15 |
| Gewindestange | 20 |
| Außengewinde | 22 |
| Handgriff | 24 |
| Adaptereinheit | 26 |
| Durchgangsöffnung | 27 |
| Innengewinde | 28 |
| Verbindungselement | 29 |
| Druckelement | 30 |
| Erster Teil | 31 |
| Zweiter Teil | 32 |
| Oberfläche | 33 |
| Hinterschneidung | 34 |
| Ausnehmung | 35 |
| Verbindungsbereich | 37 |
| Metallstab | 40 |
| Kunststoffummantelung | 41 |
| Griffteil | 50 |
| Rohr | 51 |
| Obere Fläche | 61 |
| Untere Fläche | 62 |
| Erste Flankenfläche | 63 |
| Zweite Flankenfläche | 64 |
| Gewindegang | 66 |
| Innendurchmesser | D |
| Innendurchmesser | D_{I} |
| Außendurchmesser | D_{A} |
| Länge | L |

## Patentansprüche

1. Vorrichtung (10) zum Herauspressen von Knochenzement (15) aus einer Kartusche (12), umfassend eine Gewindestange (20) mit einem Außengewinde (22), einen Handgriff (24) zum Drehen der Gewindestange (20), sowie eine Adaptereinheit (26) zum mechanischen Verbinden mit einer Kartusche (12) für Knochenzement (15), wobei die Adaptereinheit (26) eine Durchgangsöffnung (27) mit einem Innengewinde (28) für die Gewindestange (20) aufweist,
**dadurch gekennzeichnet, dass** eine äußere Oberfläche eines Gewindegangs (66) des Außengewindes (22) wenigstens 230 mm² und höchstens 370 mm² beträgt, eine Steigung des Außengewindes (22) wenigstens 1 mm und höchstens 7 mm beträgt, und ein Außendurchmesser (D_{A}) des Außengewindes (22) der Gewindestange (20) wenigstens 12 mm und höchstens 17 mm beträgt.

2. Vorrichtung (10) nach einem der vorhergehenden Ansprüche, **dadurch gekennzeichnet, dass** das Innengewinde (28) der Adaptereinheit (26) wenigstens 2 und/oder höchstens 7 Gewindegänge enthält.

3. Vorrichtung (10) nach einem der vorhergehenden Ansprüche, **dadurch gekennzeichnet, dass** das Außengewinde (22) der Gewindestange (20) eine Gewindetiefe von wenigstens 1 mm und/oder höchstens 4 mm aufweist.

4. Vorrichtung (10) nach einem der vorhergehenden Ansprüche, **dadurch gekennzeichnet, dass** die Vorrichtung (10) an dem Ende der Gewindestange (20), welches dem Handgriff (24) gegenüberliegt, ein Druckelement (30) mit einer flachen Oberfläche (33) zum direkten oder indirekten Ausüben eines Drucks auf den in der Kartusche (12) befindlichen Knochenzement (15) umfasst.

5. Vorrichtung (10) nach dem vorhergehenden Anspruch, **dadurch gekennzeichnet, dass** das Druckelement (30) zweiteilig aufgebaut ist, wobei ein erster Teil (31) des Druckelements (30) drehfest mit der Gewindestange (20) verbunden ist und ein zweiter Teil (32) des Druckelements (30) um die Längsachse der Gewindestange (20) drehbar mit dem ersten Teil verbunden ist.

6. Vorrichtung (10) nach einem der zwei vorhergehenden Ansprüche, **dadurch gekennzeichnet, dass** die Adaptereinheit (26) eine Ausnehmung (35) aufweist, wobei die Ausnehmung (35) in axialer Richtung zwischen einem Verbindungsbereich (37) oder einem Verbindungselement (29) der Adaptereinheit (26) zum mechanischen Verbinden mit der Kartusche (12) und dem Innengewinde (28) angeordnet ist, wobei die Ausnehmung (35) dazu eingerichtet ist, das Druckelement (30) zumindest im Wesentlichen aufzunehmen.

7. Vorrichtung (10) nach einem der vorhergehenden Ansprüche, **dadurch gekennzeichnet, dass** die Gewindestange (20) einen innenliegenden Metallstab (40) und eine Kunststoffummantelung (41) umfasst.

8. Vorrichtung (10) nach einem der vorhergehenden Ansprüche, **dadurch gekennzeichnet, dass** die Adaptereinheit (26) dazu eingerichtet ist, durch einen Bajonettverschluss mit der Kartusche (12) verbunden zu werden, wobei eine Drehrichtung der Adaptereinheit (26) zur Verbindung der Adaptereinheit (26) mit der Kartusche (12) einer Drehrichtung zum Einschrauben der Gewindestange (20) in die Adaptereinheit (26) entspricht.

9. Vorrichtung (10) nach einem der vorhergehenden Ansprüche, **dadurch gekennzeichnet, dass** die Gewindestange (20) eine Länge (L) zwischen 15 cm und 23 cm aufweist und/oder dass die Adaptereinheit (26) einen Verbindungsbereich (37) zum Aufstecken auf eine Kartusche (12) aufweist, wobei der Verbindungsbereich (37) einen Innendurchmesser (D) zwischen 3,3 cm und 4,0 cm aufweist.

10. Vorrichtung (10) nach einem der vorhergehenden Ansprüche, **dadurch gekennzeichnet, dass** die Adaptereinheit (26), der Handgriff (24) und/oder zumindest ein Teil der Gewindestange (20) aus Polyamid, Polyketon, Polysulfon und/oder Polyoxymethylen hergestellt ist.

11. Verwendung einer Vorrichtung (10) nach einem der vorhergehenden Ansprüche zum Herauspressen einer Menge zwischen 50 g und 130 g Knochenzement (15) aus einer Kartusche (12).

12. System zum Herauspressen von Knochenzement (15), umfassend eine Kartusche (12) für Knochenzement (15) sowie eine Vorrichtung (10) nach einem der Ansprüche 1 bis 10.

## Claims

1. An apparatus (10) for pressing out bone cement (15) from a cartridge (12), comprising a threaded rod (20) having an external thread (22), a handle (24) for rotating the threaded rod (20), and an adapter unit (26) for mechanically connecting to a cartridge (12) for bone cement (15), the adapter unit (26) comprising a passage (27) which has an internal thread (28) for the threaded rod (20),
**characterized in that** an outer surface of a thread turn (66) of the external thread (22) is at least 230 mm² and at most 370 mm², a pitch of the external thread (22) is at least 1 mm and at most 7 mm, and an outer diameter (D_{A}) of the external thread (22) of the threaded rod (20) is at least 12 mm and at most 17 mm.

2. The apparatus (10) according to any of the preceding claims, **characterized in that** the internal thread (28) of the adapter unit (26) contains at least 2 and/or at most 7 thread turns.

3. The apparatus (10) according to any of the preceding claims, **characterized in that** the external thread (22) of the threaded rod (20) has a thread depth of at least 1 mm and/or at most 4 mm.

4. The apparatus (10) according to any of the preceding claims, **characterized in that** the apparatus (10) comprises, at the end of the threaded rod (20) which is opposite the handle (24), a pressure element (30) which has a flat surface (33) for directly or indirectly exerting pressure on the bone cement (15) located in the cartridge (12).

5. The apparatus (10) according to the preceding claim, **characterized in that** the pressure element (30) is constructed in two parts, a first part (31) of the pressure element (30) being connected to the threaded rod (20) for conjoint rotation and a second part (32) of the pressure element (30) being connected to the first part for rotation about the longitudinal axis of the threaded rod (20).

6. The apparatus (10) according to either of the two preceding claims, **characterized in that** the adapter unit (26) comprises a recess (35), the recess (35) being arranged in the axial direction between a connecting region (37) or a connecting element (29) of the adapter unit (26) for mechanically connecting to the cartridge (12) and the internal thread (28), the recess (35) being designed to at least substantially accommodate the pressure element (30).

7. The apparatus (10) according to any of the preceding claims, **characterized in that** the threaded rod (20) comprises an inner metal bar (40) and a plastics casing (41).

8. The apparatus (10) according to any of the preceding claims, **characterized in that** the adapter unit (26) is designed to be connected to the cartridge (12) by a bayonet connection, with a direction of rotation of the adapter unit (26) for connecting the adapter unit (26) to the cartridge (12) corresponding to a direction of rotation for screwing the threaded rod (20) into the adapter unit (26).

9. The apparatus (10) according to any of the preceding claims, **characterized in that** the threaded rod (20) has a length (L) between 15 cm and 23 cm and/or **in that** the adapter unit (26) comprises a connecting region (37) for plugging onto a cartridge (12), the connecting region (37) having an inner diameter (D) between 3.3 cm and 4.0 cm.

10. The apparatus (10) according to any of the preceding claims, **characterized in that** the adapter unit (26), the handle (24) and/or at least part of the threaded rod (20) is made of polyamide, polyketone, polysulfone and/or polyoxymethylene.

11. A use of an apparatus (10) according to any of the preceding claims for pressing out an amount of between 50 g and 130 g of bone cement (15) from a cartridge (12).

12. A system for pressing out bone cement (15), comprising a cartridge (12) for bone cement (15) and an apparatus (10) according to any of claims 1 to 10.

## Revendications

1. Dispositif (10) permettant d'expulser par pression du ciment osseux (15) d'une cartouche (12), comprenant une tige filetée (20) comportant un filetage extérieur (22), une poignée (24) permettant de faire tourner la tige filetée (20), ainsi qu'une unité d'adaptation (26) pour la liaison mécanique avec une cartouche (12) pour du ciment osseux (15), dans lequel l'unité d'adaptation (26) présente une ouverture de passage (27) comportant un filetage intérieur (28) pour la tige filetée (20),
**caractérisé en ce qu'**une surface extérieure d'un filet de filetage (66) du filetage extérieur (22) est au moins de 230 mm² et au plus de 370 mm², un pas du filetage extérieur (22) est au moins de 1 mm et au plus de 7 mm, et un diamètre extérieur (D_{A}) du filetage extérieur (22) de la tige filetée (20) est au moins de 12 mm et au plus de 17 mm.

2. Dispositif (10) selon l'une des revendications précédentes, **caractérisé en ce que** le filetage intérieur (28) de l'unité d'adaptation (26) contient au moins 2 et/ou au plus 7 filets de filetage.

3. Dispositif (10) selon l'une des revendications précédentes, **caractérisé en ce que** le filetage extérieur (22) de la tige filetée (20) présente une profondeur de filetage d'au moins 1 mm et/ou d'au plus 4 mm.

4. Dispositif (10) selon l'une des revendications précédentes, **caractérisé en ce que** le dispositif (10) comprend, à l'extrémité de la tige filetée (20) opposée à la poignée (24), un élément de pression (30) comportant une surface plane (33) et permettant d'exercer une pression directement ou indirectement sur le ciment osseux (15) se trouvant dans la cartouche (12).

5. Dispositif (10) selon la revendication précédente, **caractérisé en ce que** l'élément de pression (30) est construit en deux parties, dans lequel une première partie (31) de l'élément de pression (30) est reliée à la tige filetée (20) de manière à ne pas pouvoir tourner et une seconde partie (32) de l'élément de pression (30) est reliée à la première partie de manière à pouvoir tourner autour de l'axe longitudinal de la tige filetée (20).

6. Dispositif (10) selon l'une des deux revendications précédentes, **caractérisé en ce que** l'unité d'adaptation (26) présente un évidement (35), dans lequel l'évidement (35) est disposé dans la direction axiale entre une zone de liaison (37) ou un élément de liaison (29) de l'unité d'adaptation (26) permettant la liaison mécanique avec la cartouche (12) et le filetage intérieur (28), dans lequel l'évidement (35) est conçu pour recevoir au moins sensiblement l'élément de pression (30).

7. Dispositif (10) selon l'une des revendications précédentes, **caractérisé en ce que** la tige filetée (20) comprend une tige métallique interne (40) et une gaine en matière plastique (41).

8. Dispositif (10) selon l'une des revendications précédentes, **caractérisé en ce que** l'unité d'adaptation (26) est conçue pour être reliée à la cartouche (12) au moyen d'une fermeture à baïonnette, dans lequel un sens de rotation de l'unité d'adaptation (26) pour la liaison de l'unité d'adaptation (26) à la cartouche (12) correspond à un sens de rotation pour le vissage de la tige filetée (20) dans l'unité d'adaptation (26).

9. Dispositif (10) selon l'une des revendications précédentes, **caractérisé en ce que** la tige filetée (20) présente une longueur (L) comprise entre 15 cm et 23 cm **et/ou en ce que** l'unité d'adaptation (26) présente une zone de liaison (37) permettant l'emboîtement sur une cartouche (12), dans lequel la zone de liaison (37) présente un diamètre intérieur (D) compris entre 3,3 cm et 4,0 cm.

10. Dispositif (10) selon l'une des revendications précédentes, **caractérisé en ce que** l'unité d'adaptation (26), la poignée (24) et/ou au moins une partie de la tige filetée (20) sont fabriquées en polyamide, polycétone, polysulfone et/ou polyoxyméthylène.

11. Utilisation d'un dispositif (10) selon l'une des revendications précédentes pour l'expulsion par pression d'une quantité comprise entre 50 g et 130 g de ciment osseux (15) hors d'une cartouche (12).

12. Système permettant l'expulsion par pression de ciment osseux (15), comprenant une cartouche (12) pour du ciment osseux (15) ainsi qu'un dispositif (10) selon l'une des revendications 1 à 10.
